# EUROPEAN PATENT SPECIFICATION

(11) **EP 4 583 772 B1**
(45) Date of publication and mention of the grant of the patent: **18.02.2026**
(21) Application number: 22777738.0
(22) Date of filing: 09.09.2022
(51) Int. Cl.: A61B 5/097, A61B 5/08

(54) **DEVICE FOR COLLECTING PARTICLES IN AN EXHALED AIRFLOW AND METHOD FOR EXTRACTING COLLECTED PARTICLES**
VORRICHTUNG ZUM SAMMELN VON PARTIKELN IN EINEM AUSGEATMETEN LUFTSTROM UND VERFAHREN ZUR EXTRAKTION GESAMMELTER PARTIKEL
DISPOSITIF DE COLLECTE DE PARTICULES DANS UN FLUX D'AIR EXPIRÉ ET PROCÉDÉ D'EXTRACTION DE PARTICULES COLLECTÉES

(43) Date of publication of application: 16.07.2025
(73) Proprietor: Munkplast AB, 752 28 Uppsala (SE)
(72) Inventor: STAMBECK, Peter, 743 71 Björklinge (SE)
(74) Representative: Bergenstråhle & Partners AB
(86) International application number: PCT/SE2022/050801
(87) International publication number: WO 2024/054139

(56) References cited:
- US-A1- 2005 137 491
- US-A1- 2019 170 629
- US-A1- 2019 336 799
- US-A1- 2021 137 415

## Description

### Technical Field

The present invention relates generally to a device for collecting aerosol particles in an exhaled airflow. Said particles may be aerosol particles consisting mainly of surfactant formed or found in the alveoli of the lungs, such as biomarkers or exogenous compounds containing traces of drugs or other substances.

### Background Art

Human breath contains aerosol particles that are formed from the respiratory tract lining fluid covering the airways during normal breathing. Said particles have a size of between 0.1 and 2 µm, with an average size of between 0.3 and 0.8 µm. See article Characterization of Exhaled particles from the Healthy Human Lung, Journal of aerosol medicine and pulmonary drug delivery, Volume 23, Number 6, 2010 by Schwarz et al. The aerosol particles carry non-volatile components containing diagnostic information or biomarkers and are often studied as the breath condensate fraction. In this aerosol fraction, both lipids and peptides of endogenous origin have been demonstrated. It has also been discovered that exogenous compounds are present in the exhaled breath. Such exogenous compound may for example be drugs and narcotics. The respiratory tract lining fluid contain large quantities of antioxidants and surfactant. The surfactant phase is lipophilic and may represent a compartment for the exogenous compounds. Thus, exhaled breath can be used as a matrix for several types of testing such as for example testing of a medical condition or a medical treatment procedure, abused drug testing or doping testing. It can also be used for medical research.

With the discovery of exogenous aerosol particles consisting mainly of surfactant present in exhaled breath, a need for new methods and devices for collecting and analyzing said surfactant aerosol particles in exhaled breath has arisen. For accurate analysis it is of importance that as many of the aerosol particles as possible is collected from a sample breath. Further, in some applications, such as for example testing for drug abuse or doping, the collection of particles is performed away from a lab environment.

WO 2017/001217 A1, discloses a collecting device having a labyrinth or zigzag-shaped flow path formed by baffles in an elongated housing which forces the exhaled flow of air to change direction multiple times. The change of direction of the airflow separates the heavier aerosol particles in the exhaled air from the air itself. The heavier particles continue in the original flow direction and collide with and attaches to the baffles or the inner wall of the housing, while the air changes direction and follow the labyrinth-shaped flow path.

WO 2017/091134 A1, discloses a portable sampling device for use with one or more collecting devices in accordance with WO 2017/001217 A1. After a sample has been proved by a user, the collecting device is removed from the sampling device and sent to e.g. a laboratory to extract the collected particles from the collecting device. To this end, the collecting device is inserted into a test tube or vial and an amount of a solvent called an eluent is added to release the particles through the process of elution, i.e. washing. The eluent fluid is selected depending on the adsorption strength of the analyte (collected particles), the desired eluting power of the eluent and the effect on the analyte so as not to destroy the particles. Typically, the eluent is acetone, methanol, ethanol or water.

Further devices and methods for collecting aerosol particles are known from US2021/137415-A1, US2019/170629-A1, US2005/137491-A1 and US2019/336799-A1.

Eluent fluid can be expensive which drives cost in cases where a great number of samples are to be analysed. Evaporating the resulting volume of eluate fluid is also time consuming, limiting the throughput and capacity of analysis. Thus, there is a need to find improved solutions which reduce the cost and time of analysis.

### Summary of Invention

An objective of the present invention is to provide a small and simple but yet reliable and effective device for collecting aerosol particles, preferably aerosol particles consisting mainly of surfactant functioning as biomarkers, in exhaled breath, and which facilitates subsequent extraction of the collected particles. The device is described in the appended claims.

According to a first aspect of the present disclosure, there is provided a device for collecting aerosol particles in an exhaled airflow, said device comprising: a housing extending in a longitudinal direction between a first end with an inlet and a second end with an outlet, and an inner cross-section defined by an inner circumferential wall of the housing, wherein the cross-section exhibits a transverse width, and at least four transverse baffles, arranged at a distance from each other and extending in a direction substantially perpendicular to the walls, partly covering the inner cross section of the housing, wherein said transverse baffles protrude from opposite sides of the inner wall of the housing to create a zigzag-shaped flow path from said inlet to said outlet. Each transverse baffle has at least one throughgoing opening, wherein the openings are substantially aligned with each other to form a substantially longitudinal passage through the housing.

The longitudinal passage provides a conduit for introducing a needle into the collecting device to deliver an eluent fluid directly to the interior of the housing. Thus the passage enables improved distribution of eluent fluid to efficiently wash the collected particles from the inner wall and baffles of the collecting device. Thereby, the amount of eluent fluid required for the elution process can be decreased to reduce the cost and time of the analysis. The openings can be formed during or after manufacturing of the collecting device, for instance using a cylindrical positive die form during moulding or by cutting the openings with a suitable tool after moulding of the housing.

In one embodiment, the openings are formed by a slot extending from a free end of each transverse baffle. The slots may easily be formed in the transverse baffles during the manufacturing process.

In one embodiment, only a portion of each opening is aligned with each other. In this way, the cross-sectional area of the passage can be made smaller than the openings.

In one embodiment, the ratio between the cross-sectional area of the passage and the inner cross-sectional area of the housing is equal to or greater than 1:5, preferably 1:10, most preferably 1:100. By making the passage and openings small in relation to the inner cross-section of the housing, most of the exhaled airflow will follow the zigzag-shaped flow path created by the transverse baffles, whilst only a fraction will pass through the openings in each transverse baffle.

In one embodiment, the passage is substantially straight and extends from the first end to the second end of the housing. This configuration allows insertion of the needle through the whole housing of the device to reach each baffle.

In one embodiment, the housing comprises a first and a second housing half which are adapted to be joined. When the device is designed to be constructed out of two, preferably identical, halves the production process is simplified. By dividing the device in two, each transverse baffle with the openings and possible rough structure on the inner wall can be easily produced by using a cutting production tool. It is also possible to produce two identical halves of which one is turned 180 degrees before joining.

In one embodiment, the passage is coaxial with the housing. By letting the passage and the housing have a common axis, the passage will extend through the centre of the housing to ensure optimal distribution of the eluent fluid with the needle.

In a second aspect of the present disclosure, there is provided a method for method of extracting collected particles from a collecting device according to the first aspect, comprising the steps: placing the collecting device in a receptacle; introducing a needle into the collecting device through the longitudinal passage formed by the openings; and delivering an eluent fluid to the interior of the collecting device through the needle.

In one embodiment, the needle comprises at least one lateral opening for lateral delivery of the eluent fluid. By delivering the eluent fluid laterally, the inner wall and transverse baffles may be sprayed directly to improve extraction of the collected particles.

In one embodiment, said particles in exhaled airflow are biomarkers, such as pulmonary surfactant, drugs or other endogenous or exogenous compounds found in the alveoli of the lungs having a size of between 0.1 and 2 µm, with an average size of between 0.3 and 0.8 µm.

Aerosol particles in the form of biomarkers or particles related to drugs or other exogenous compounds are the most interesting particles arranged to be collected by this device. Said particles have an average size of between 0.3 and 0.8 µm. Larger particles such as saliva or other particles will to a large extent be collected on the transverse baffle which is arranged closest to the inlet, i.e. closest to the mouth of the person exhaling into the device. Smaller and lighter particles will to a large extent follow the airflow and exit the device through the outlet.

### Brief Description of Drawings

The invention is now described, by way of example, with reference to the accompanying drawings, in which:
Fig. 1 is a cross-sectional view of a collecting device illustrating the working principle of collecting aerosol particles in exhaled breath;
Fig. 2a is an end view of the collecting device of Fig. 1;
Fig. 2b is an end view of an alternative collecting device;
Fig. 3 is a perspective view of a collecting device according to one embodiment of the present disclosure;
Fig. 4 is a perspective view of one half of a collecting device according to one embodiment of the present disclosure;
Fig. 5 is an end view of a collecting device according to one embodiment of the present disclosure;
Fig. 6 is an end view of one half of a collecting device according to one embodiment of the present disclosure;
Fig. 7 is a cross-sectional view of a collecting device according to one embodiment of the present disclosure; and
Fig. 8 is a cross-sectional view of a collecting device according to one embodiment of the present disclosure during delivery of eluent fluid.

### Description of Embodiments

In the following, a detailed description of device according to the invention is presented. In the drawing figures, like reference numerals designate identical or corresponding elements throughout the several figures. It will be appreciated that these figures are for illustration only and do not in any way restrict the scope of the invention.

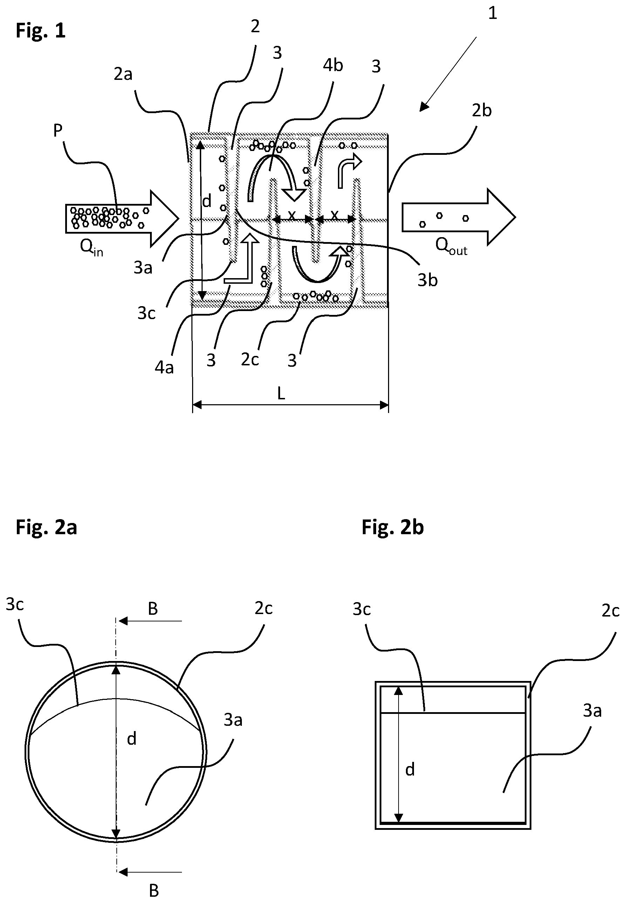

Figs. 1 and 2a-2b disclose a device 1 for collection particles in exhaled breath. Fig. 1 is a cross-sectional view taken at cut B-B in Fig. 2a and shows the inside of the device 1.

The device 1 comprises a housing 2 having a length L in a longitudinal extension direction between a first end 2a with an inlet and a second end 2b with an outlet. The inlet is arranged to receive an exhaled airflow Qᵢₙ comprising aerosol particles P from a subject, such as for example a person, and the outlet is arranged to transmit the exhaled airflow Qₒᵤₜ from the device 1. Thus, the exhaled air is arranged to flow in a direction from the inlet to the outlet. The housing 2 has an inner cross-sectional area defined by an inner circumferential wall 2c of the housing 2. In the embodiment shown in Figs. 1 and 2a the housing has an elongated cylindrical shape with a length L and a circular cross-section, i.e. the cross-sectional area is defined by the transverse width equal to the inner diameter d of the housing 2. Fig. 2b discloses an alternative device where the housing 2 has a rectangular cross-section and the cross-sectional area is defined by the height and transverse width d of the housing. Other cross-sectional area shapes are also possible but the cross-sectional area is always defined by the transverse width d which is equal to the distance between opposite inner walls 2c of the housing.

The outer diameter of the housing is in one embodiment of such a dimension that it can easily be fitted into a standard size test tube. I.e. it has a diameter between 8 and 30 mm, preferably between 10 and 20 mm. Said inner cross-sectional area is therefore slightly less than the area given by the outer diameter, depending on the thickness of the housing wall 2c. Therefore said cross-sectional area may be between 20 mm² and 615 mm², preferably between 50 and 250 mm², most preferably between 70 and 90 mm². Comparably, said distance d between the inner walls 2c of the housing may be between 5 and 28 mm, preferably between 8 and 18 mm, most preferably between 9,5 and 10,5 mm.

At least four transverse baffles 3 in the form of partition walls are arranged to extend from the inner wall 2c in a substantially perpendicular direction, thus substantially perpendicular to the initial direction of the exhaled airflow when exiting the subject's mouth. Each transverse baffle 3 has a first surface 3a facing the airflow, an opposite second surface 3b and a peripheral edge or free end 3c. The first and second surface 3a and 3b each have a surface area smaller than the inner cross-sectional area of the housing 2. Thus, the transverse baffles have a surface area partly covering the inner cross-sectional area of the housing 2. In different embodiments the transverse baffles have a surface area covering 50-95%, preferably 60-85%, more preferably 65-80% of the inner cross-sectional area of the housing 2.

The transverse baffles 3 protrude from opposite sides of the inner wall 2c of the housing 2. Thus, the baffles create opposite openings 4a, 4b between the transverse baffles 3 and the housing inner wall 2c leaving an opening area equal to the difference between the inner cross-sectional area of the housing 2 and the surface area of the transverse baffle 3.

Said transverse baffles 3 are arranged to create a zigzag or labyrinth-shaped flow path having a cross-sectional flow area from said inlet to said outlet. When the airflow collides with a surface substantially perpendicular to the airflow, the flow is diverted in a direction parallel to said surface. Said diversion of the airflow separates the heavier particles P in the exhaled air from the air itself. The heavier particles P continue in the original flow direction and collide with the transverse baffles 3 or the inner wall 2c of the housing 2, while the air changes direction and follows the zigzag-shaped flow path. The longer distance the airflows and the more and larger direction changes the airflow is forced to do, the larger amount of particles is separated from the air and collected in the device 1. Further, a direction change also creates a turbulent flow during which the particles are more easily separated from the air. A turbulent airflow also increases the impact frequency between the particles and the surfaces of the walls of the device 1, thus increasing the amount of airborne particles P attaching to the surfaces. As a result, the outflow Qₒᵤₜ out of the device 1 comprises less particles P than the inflow Qᵢₙ into the device 1.

A person is only able to exhale with a certain maximum flow rate Qᵢₙ. At a certain counter pressure from the device the velum of the person closes and exhalation is impossible. The pressure difference over the device must therefore not be too high. However, a certain inflow Qᵢₙ and pressure difference is necessary to create the certain conditions with a high enough flow velocity to separate the particles from the airflow. It is therefore important to design the device to have a certain flow path cross-sectional flow area which is defined by a first cross-sectional flow area, defined by the opening area between the transverse baffles 3 and the inner wall 2c of the housing 2 and a second cross-sectional flow area delimited by the transverse baffles 3 and the inner diameter of the housing 2. The parameters defining the second cross-sectional flow area are the specific extension length L of the housing, the transverse width d of the housing 2 or inner diameter in the case of a cylindrical housing, and the number of transverse baffles 3 of the device 1, more particularly, the distance x between the transverse baffles 3. The opening area mentioned above is preferably within the interval of 10 mm² - 25 mm², said extension length L between 10 and 70 mm and the number of transverse baffles 3 between four and fourteen. The first cross-sectional flow area is in one embodiment smaller than the second cross-sectional flow area. This relationship increases the acceleration of the air flowing past the peripheral edge 3c of the transverse baffle 3.

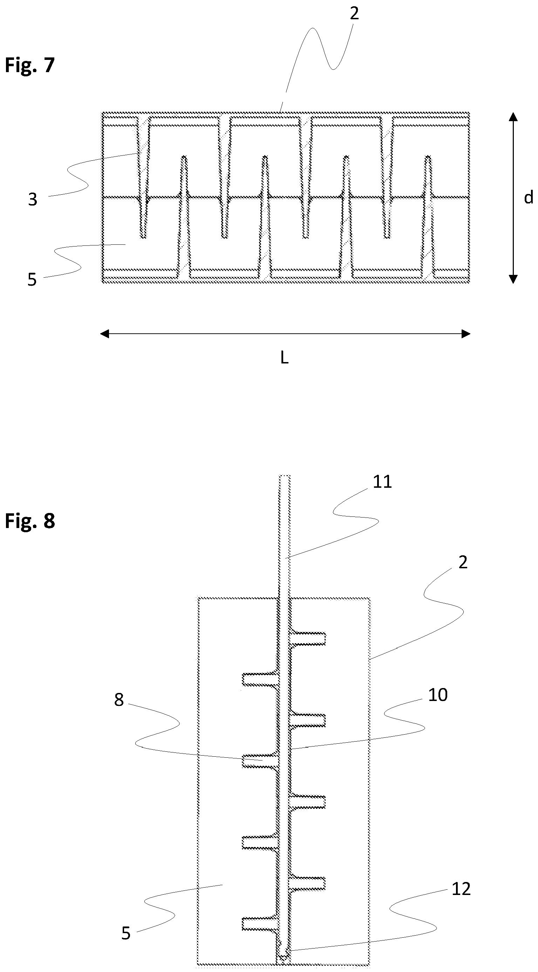

In the embodiment shown for example in Fig. 7, the number of transverse baffles 3 are eight, the length L approximately 22 mm and the opening area is approximately 20 mm². Thus, in this embodiment the wall surface area A2 covers approximately 75% of the total inner cross-sectional area.

In order to increase the flow area, it is in one embodiment possible to arrange more than one devices parallel to each other in an additional outer housing (not shown) thus decreasing the total flow resistance.

The transverse baffles 3 may be separated from each other with a certain distance x depending on the maximum allowed pressure difference over the device. The distance x depends on the length L of the device and the number of transverse baffles 3. However, in order to create the certain conditions with a high enough flow velocity to separate the aerosol particles from the airflow, the distance x between at least two opposite transverse baffles 3 is always smaller than the distance d between the inner walls of the housing. In one embodiment, shown in Fig. 1 the distance x is constant. Alternatively, the distance between adjacent transverse baffles 3 increases in the direction from the first end 2a towards the second end 2b of the device 1. With an increasing distance between the transverse baffles 3 the flow velocity through the device 1 can be substantially maintained. The second cross-sectional flow area increases towards the outlet 2b due to the increasing distance between the transverse baffles 3 while the first cross-sectional flow area equal to the opening area between the transverse baffles 3 and the inner wall 2c is kept constant. However, it is also possible to gradually increase the opening area in the direction from the first end 2a towards the second end 2b of the device 1. This will further decrease the flow resistance in the device and contribute to a maintained flow velocity. In one embodiment, the relation between the first flow path cross-sectional flow area and the second flow path cross-sectional flow area is kept substantially constant throughout the entire length L of the device.

The device is in one embodiment made of a non-absorbent material, for example polymer materials such as for example polypropylene (PP), polyvinylidene fluoride (PVDF), polytetrafluoroethylene (PTFE), fluorinated ethylene propylene (FEP) or other non-absorbent, preferably, polymer materials. On a non-absorbent material the particles may attach but are easily washed off when a later analysing step is performed. The washing off may be performed in a test tube filled with an amount of test fluid enough to cover the entire length L of the housing.

In order to increase the surface area on which the particles P are collected, in one embodiment the inner wall 2c of the housing 2 has a rough surface structure. The rough structure may be adapted to the size of the particles to be collected. I.e. if the aerosol particles to be collected have a diameter of 0.1 and 2.0 µm, the inner wall 2c may preferably be provided or covered with cavities of approximately the same size. The surface may also be machined to have protrusions distanced by approximately the same distance as the diameter of the particles. Said rough structure may also for example be a spark or electro eroded surface with a surface roughness Ra from 0.1 micron to up to 12.5 micron. Said possible surface roughness value also depend on the draft angle on the surface to be eroded in relation to the tool producing the eroded surface. With a larger draft angle a larger surface roughness is possible to create.

It is also possible to further increase the surface area by providing all surfaces of the device, both inner and outer with a rough structure. Different surface roughness values are possible on different surfaces of the device 1.

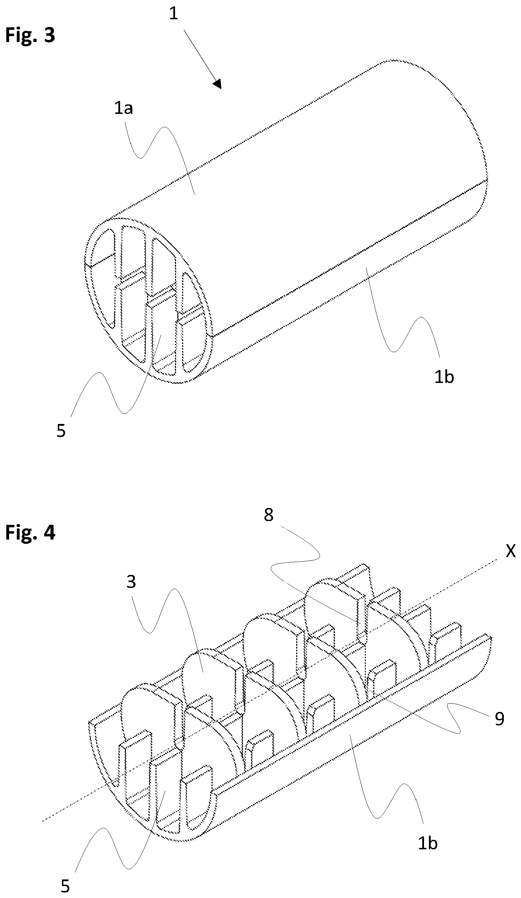

In one embodiment the first surface 3a and second surface 3b of the transverse baffles 3 are oriented at an angle in relation to each other such that the edge 3c of the respective transverse baffle 3 is narrower than the base of the transverse baffle 3. However, the first surface 3a and second surface 3b of the transverse baffles 3 may also be parallel or substantially parallel to each other, as can been seen in Fig. 4. The may also be produced having only a small draft angle.

Referring now to Figs. 3 and 4, the device 1 is shown comprising two housing halves 1a, 1b adapted to be joined together to form the housing 2, here in the form of an elongated cylinder. Each housing half 1a, 1b comprises at least two transverse baffles 3 extending further than half of the transverse width d of the housing 2. In one embodiment the transverse baffles 3 extend between 10% and 60% further than half of the transverse width d of the housing 2.

Each housing half 1a, 1b may comprise at least one longitudinal baffle 5 arranged substantially parallel to the longitudinal extension direction of the housing 1 and extending a maximum of half the transverse width d of the housing 1. In order for the transverse baffles 3 to be able to extend further than half of the transverse width d of the housing 2, the longitudinal baffles 5 have recesses 9 as may be seen in Fig. 4, which are adapted to receive the edges 3c of the transverse baffles 3. The transverse baffles 3 are in one embodiment arranged to be inserted by press fitting into the recesses 9. A press fit also has the advantage of acting like a glue, joining the two housing halves 1a and 1b.

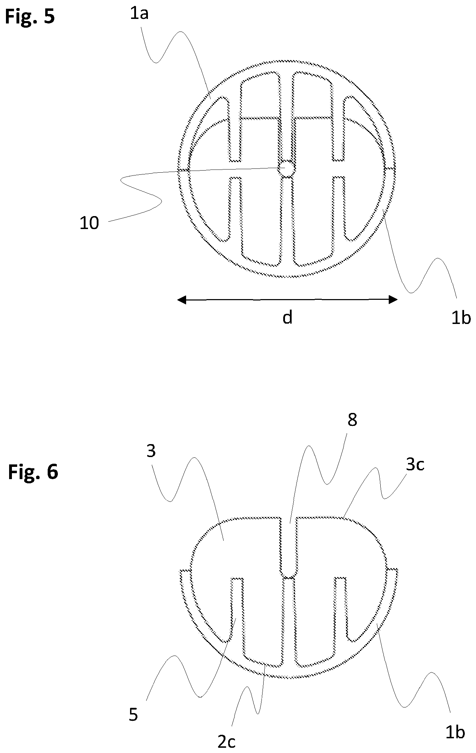

Referring now to Figs. 4-6, each of the transverse baffles 3 comprises a through-going opening 8. The openings 8 are substantially aligned with each other in the longitudinal direction of the device 1 to form a substantially straight longitudinal passage 10 extending through the device 1 as may be seen in Fig. 5. The passage 10 extends substantially through the whole housing 2, from the first end 2a to the second end 2b. In one embodiment, the openings 8 are formed by cut-out portions or slots extending from the edge 3c of the transverse baffles 3 towards the inner wall 2c of the housing 2. In this case, only a portion of each opening 8 is aligned with each other due to the alternating transverse baffles 3 protruding from opposite sides of the inner wall 2c. As a result, the cross-sectional area of the passage 10 is smaller than each opening 8. In one embodiment, the passage 10 is coaxial with the housing 2, i.e. extending along the central longitudinal axis X of the housing 2.

Referring now to Fig. 8, there is shown a cross-sectional view of the device 1 showing the interior of the housing 2. The section is taken through a central longitudinal baffle 5, thus not showing the transverse baffles 3 but only the openings 8 therein. The longitudinal passage 10 provides a conduit for introducing a needle 11 into the collecting device 1 to deliver an eluent fluid directly to the interior of the housing 2. Thus the passage 10 enables improved distribution and delivery of eluent fluid to efficiently wash the collected particles P from the inner wall 2c and baffles 3, 5 of the collecting device 1. Thereby, the amount of eluent fluid required for the elution process can be decreased to reduce the cost of the analysis. In one embodiment, the needle comprises one or more lateral openings such that the eluent fluid may be delivered laterally in a radial direction towards the inner wall 2c.

In a method of the present disclosure, a breath sample from a subject has been collected using the device 1. The device 1 is then placed in a suitable receptacle for extracting the collected particles P through elution. In the case of a collecting device 1 shaped like an elongated cylinder, the receptacle may be a test tube or vial or similar. A needle of syringe containing the eluent fluid is then introduced into the longitudinal passage 10, and the eluent fluid is delivered to the interior of the housing 2 of the device 1 by depressing a plunger of the syringe. The eluent fluid washes the collected particles P from the interior surface of the housing 2 and flows towards the bottom of the test tube. The thus accumulated eluate will then have a higher concentration of aerosol particles compared to a method where a higher amount of eluent fluid is used.

Embodiments of a device for collecting particles according to the present disclosure has been described. However, the person skilled in the art realises that this can be varied within the scope of the appended claims without departing from the inventive idea.

All the described alternative embodiments above or parts of an embodiment can be freely combined without departing from the inventive idea as long as the combination is not contradictory.

## Claims

1. A device (1) for collecting aerosol particles in an exhaled airflow, said device comprising:
- a housing (2) extending in a longitudinal direction between a first end (2a) with an inlet and a second end (2b) with an outlet, and an inner cross-section defined by an inner circumferential wall (2c) of the housing, wherein the cross-section exhibits a transverse width (d), and
- at least four transverse baffles (3), arranged at a distance from each other and extending in a direction substantially perpendicular to the inner wall, partly covering the inner cross section of the housing, wherein said transverse baffles protrude from opposite sides of the inner wall of the housing to create a zigzag-shaped flow path from said inlet to said outlet,
**characterised in that** each transverse baffle has at least one throughgoing opening (8), wherein the openings are substantially aligned with each other to form a substantially longitudinal passage (10) through the housing.

2. The device according to claim 1. wherein the openings are formed by a slot extending from a free end (3c) of each transverse baffle.

3. The device according to claim 1 or 2, wherein only a portion of each opening is aligned with each other.

4. The device according to any one of the preceding claims, wherein the ratio between the cross-sectional area of the passage and the inner cross-sectional area of the housing is equal to or greater than 1:5, preferably 1:10, most preferably 1:100.

5. The device according to any one of the preceding claims, wherein the passage is substantially straight and extends from the first end to the second end of the housing.

6. The device according to any one of the preceding claims, wherein the housing comprises a first and a second housing half (1a, 1b) which are adapted to be joined.

7. The device according to any one of the preceding claims, wherein the passage is coaxial with the housing.

8. A method of extracting collected particles from a collecting device (1) according to any one of the preceding claims, comprising the steps:
- placing the collecting device in a receptacle;
- introducing a needle (11) into the collecting device through the longitudinal passage (10) formed by the throughgoing openings (8); and
- delivering an eluent fluid to the interior of the collecting device (1) through the needle.

9. The method according to claim 8, wherein the needle comprises at least one lateral opening (12) for lateral delivery of the eluent fluid.

## Patentansprüche

1. Vorrichtung (1) zum Sammeln von Aerosolteilchen in einem ausgeatmeten Luftstrom, wobei die Vorrichtung Folgendes umfasst:
- ein Gehäuse (2), das sich in einer Längsrichtung zwischen einem ersten Ende mit einem Einlass (2a) und einem zweiten Ende mit einem Auslass (2b) erstreckt, und ein innerer Querschnitt durch eine innere Umfangswand (2c) des Gehäuses definiert ist, wobei der Querschnitt eine Querbreite (d) aufweist; und
- mindestens vier quer verlaufende Leitbleche (3), die in einem Abstand voneinander angeordnet sind und sich in eine Richtung erstrecken, die im Wesentlichen senkrecht zur Innenwand ist, den inneren Querschnitt des Gehäuses teilweise bedeckend, wobei die quer verlaufende Leitbleche von gegenüberliegenden Seiten der Innenwand des Gehäuses hervorstehen, um einen zickzackförmigen Strömungsweg vom Einlass zum Auslass zu bilden,
**dadurch gekennzeichnet, dass** jedes quer verlaufende Leitblech mindestens eine durchgehende Öffnung (8) aufweist, wobei die Öffnungen zueinander im Wesentlichen ausgerichtet sind um einen im Wesentlichen längsverlaufenden Durchgang (10) durch das Gehäuse zu bilden.

2. Vorrichtung nach Anspruch 1, wobei die Öffnungen von einem Schlitz gebildet sind, der sich von einem freien Ende (3c) jedes quer verlaufenden Leitbleches erstreckt.

3. Vorrichtung nach Anspruch 1 oder 2, wobei nur ein Teil jeder Öffnung zueinander ausgerichtet ist.

4. Vorrichtung nach einem der vorhergehenden Ansprüche, wobei das Verhältnis zwischen der Querschnittsfläche des Durchgangs und der inneren Querschnittsfläche des Gehäuses gleich oder größer als 1:5, vorzugsweise 1:10, am meisten bevorzugt 1:100 ist.

5. Vorrichtung nach einem der vorhergehenden Ansprüche, wobei der Durchgang im Wesentlichen gerade ist und sich vom ersten Ende zum zweiten Ende des Gehäuses erstreckt.

6. Vorrichtung nach einem der vorhergehenden Ansprüche, wobei das Gehäuse eine erste und eine zweite Gehäusehälfte (1a, 1b) umfasst, die zum Zusammenfügen angepasst sind.

7. Vorrichtung nach einem der vorhergehenden Ansprüche, wobei der Durchgang koaxial mit dem Gehäuse verläuft.

8. Verfahren zum Entnehmen gesammelter Partikel aus einer Sammelvorrichtung (1) nach einem der vorhergehenden Ansprüche, umfassend die folgenden Schritte:
- Platzieren der Sammelvorrichtung in einem Behälter;
- Einführen einer Nadel (11) in die Sammelvorrichtung durch den durch die durchgehenden Öffnungen (8) gebildeten Längskanal (10); und
- Zuführen einer Elutionsflüssigkeit in das Innere der Sammelvorrichtung (1) durch die Nadel.

9. Verfahren nach Anspruch 8, wobei die Nadel mindestens eine seitliche Öffnung zur seitlichen Zuführung der Elutionsflüssigkeit umfasst.

## Revendications

1. Dispositif (1) pour collecter des particules d'aérosol dans un flux d'air expiré, comprenant :
- un boîtier (2) s'étendant dans une direction longitudinale entre une première extrémité (2a) avec une entrée et une seconde extrémité (2b) avec une sortie, et présentant une section transversale interne définie par une paroi circonférentielle interne (2c) du boîtier, cette section transversale ayant une largeur (d) ;
- au moins quatre déflecteurs transversaux (3), disposés à distance les uns des autres et s'étendant sensiblement perpendiculairement à la paroi interne, recouvrant partiellement la section transversale interne du boîtier, dans lequel lesdits déflecteurs transversaux font saillie de part et d'autre de la paroi interne du boîtier afin de créer un trajet d'écoulement en forme zigzag depuis l'entrée vers la sortie,
**caractérisé en ce que** chaque déflecteur transversal présente au moins une ouverture traversante (8), dans lequel les ouvertures sont sensiblement alignées entre elles pour former un passage sensiblement longitudinal (10) à travers le boîtier.

2. Dispositif selon la revendication 1, dans lequel les ouvertures sont formées par une fente s'étendant depuis une extrémité libre (3c) de chaque déflecteur transversal.

3. Dispositif selon la revendication 1 ou 2, dans lequel seule une partie de chaque ouverture est alignée entre elles.

4. Dispositif selon l'une quelconque des revendications précédentes, dans lequel le rapport entre la section transversale du passage et la section transversale intérieure du boîtier est égal ou supérieur à 1:5, de préférence 1:10, et idéalement 1:100.

5. Dispositif selon l'une quelconque des revendications précédentes, dans lequel le passage est sensiblement rectiligne et s'étend de la première extrémité à la seconde extrémité du boîtier.

6. Dispositif selon l'une quelconque des revendications précédentes, dans lequel le boîtier comprend une première et une seconde moitié (1a, 1b) adaptées pour être assemblées.

7. Dispositif selon l'une quelconque des revendications précédentes, dans lequel le passage est coaxial au boîtier.

8. Procédé d'extraction de particules collectées à partir d'un dispositif de collecte (1) selon l'une quelconque des revendications précédentes, comprenant les étapes suivantes :
- placer le dispositif de collecte dans un récipient ;
- introduire une aiguille (11) dans le dispositif de collecte par le passage longitudinal (10) formé par les ouvertures traversantes (8) ; et
- acheminer un fluide éluant à l'intérieur du dispositif de collecte (1) par l'intermédiaire de l'aiguille.

9. Procédé selon la revendication 8, dans lequel l'aiguille comprend au moins une ouverture latérale (12) pour l'acheminement latéral du fluide éluant.
